# EUROPEAN PATENT SPECIFICATION

(11) **EP 4 591 755 B1**
(45) Date of publication and mention of the grant of the patent: **07.10.2026**
(21) Application number: 25153541.5
(22) Date of filing: 23.01.2025
(51) Int. Cl.: A45D 40/00

(54) **COSMETIC ARTICLE**
KOSMETIKARTIKEL
ARTICLE COSMÉTIQUE

(30) Priority: 25.01.2024 IT 202400001419
(43) Date of publication of application: 30.07.2025
(73) Proprietor: Chromavis S.p.A., 26010 Offanengo (CR) (IT)
(72) Inventor: Valvassori, Sergio, 26010 Ripalta Cremasca (CR) (IT); Uggè, Martina, 26012 Castelleone (CR) (IT); Faure Beryl Laurene, Magali, 26013 Crema (CR) (IT)
(74) Representative: Vanosi, Adelio Valeriano

(56) References cited:
- EP-A1- 3 738 478
- WO-A1-2021/244954
- FR-B1- 2 890 952
- US-A- 4 884 719
- US-A- 5 566 829
- US-A1- 2021 188 523

## Description

### FIELD OF THE INVENTION

The present invention relates to a cosmetic article, in particular for make-up.

In particular, the invention refers to a cosmetic article comprising a make-up product made in the form of a strip wound on a spool.

EP 3 738 478 A1 and US 5 566 829 A describe devices known in the art.

### BACKGROUND ART

In the cosmetic field, and especially in the field of make-up articles, there is an ongoing search for new product configurations that are attractive, practical to use, and innovative for the public.

### SUMMARY OF THE INVENTION

The object of this invention is to provide a cosmetic article that makes a make-up product available in a new form, which is flexible so as to be easily dispensed by end users.

A further object of the present invention is to make available a refillable, and therefore environmentally friendly, cosmetic article that can be dispensed in the correct amount.

This and other objects are achieved by means of a cosmetic article produced according to the technical teachings of the claims annexed hereto.

### BRIEF DESCRIPTION OF THE FIGURES

Further characteristics and advantages of the invention will become clearer in the description of a preferred but not exclusive embodiment of the cosmetic article, illustrated - by way of a non-limiting example - in the drawings annexed hereto, in which:
Figure 1 is a perspective view of the cosmetic article according to the present invention, in the open position;
Figure 2 is the perspective view of the article in Figure 1, in the closed position;
Figure 3 is an exploded view of the cosmetic article according to the present invention;
Figure 4 is a lateral section view of the cosmetic article of Figure 1, in an open position;
Figure 5 is a section view of Figure 4, with the article in a position immediately prior to a cutting position; and
Figure 6 is the section view of Figure 4 with the article in the closed position.

### DETAILED DESCRIPTION OF THE INVENTION

With reference to the figures stated, reference number 2 is used to denote, as a whole, a cosmetic article, preferably a make-up article.

The cosmetic article 2 comprises a cosmetic product 1, preferably for make-up, in the form of a flexible strip 2A (or tape) wound on a spool 3, a base 4 that rotatably supports the spool, and a lid 6 that, when in a closed position, protects the spool 3 on which the cosmetic product 1 is wound.

Advantageously, the article comprises a cutter 5 for cutting part of the strip 2A of cosmetic product 1.

The cutter 5 can be coupled with the lid 6.

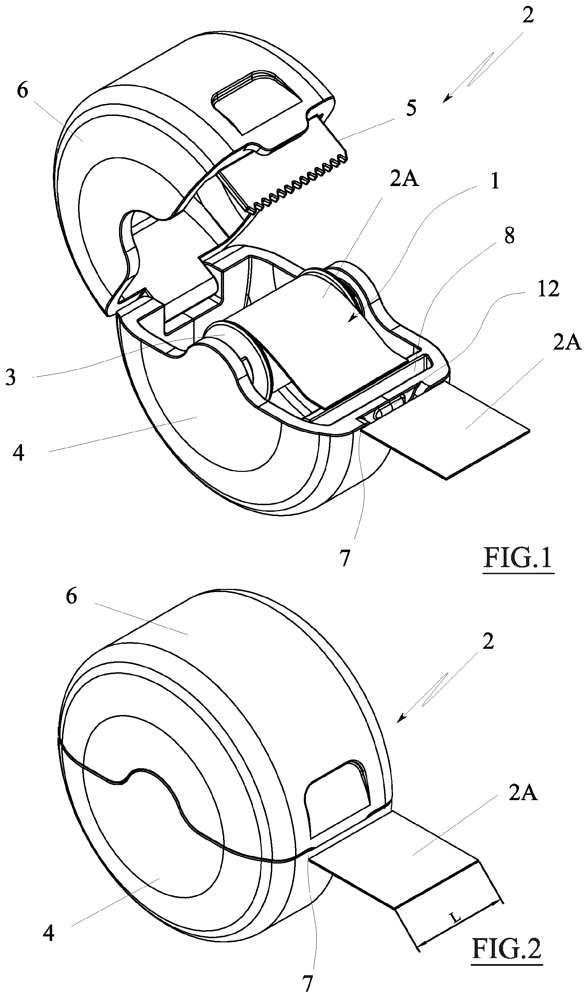

As can be seen, for example in Figure 2, the base 4 can determine a surface or plane 7 on which one end of the strip 2A simply rests, so that (after cutting) a front part of the strip 2A can rest on the surface 7 or that (before cutting) a part of the strip 2A protrudes from the surface 7 as shown in Figure 1.

Obviously, the surface 7 can, if necessary, be replaced by a window or slot through which the end of the strip 2A can fit.

Furthermore, between the surface 7 and the spool 3, the base 4 can feature a cavity 8, with essentially the same conformation as the said cutter 5, into which the said cutter 5 fits upon closure of the lid 6, thereby cutting the strip 2A when the said strip 2A is resting on the surface 7, protruding from the base 4 (as in Figure 1).

To facilitate both the cutting action and usability of the article, the lid 6 can be hinged onto the base 4.

The spool 3 can be removably coupled with the base 4 so as to be replaced or reloaded once the original spool is empty.

For this purpose, the base 4 can feature at least a pair of cradle seats 9 that house corresponding pins 10 that extend from the spool 3, which features, in proximity to each of the said pins 10, a flange 11 that limits lateral movement of the strip 2A when the spool rotates around the said pins 10.

Furthermore, snap means 12 can be envisaged between the lid 6 and the base 4, which can be released (either automatically, by simply pulling, or by pressing a suitable release button not shown) and which are configured to keep the lid 6 closed at least when the cosmetic article 1 is not in use.

As can be seen in Figure 2, when the lid is closed, the cosmetic article can have an essentially cylindrical configuration, similar to that of a thick disk.

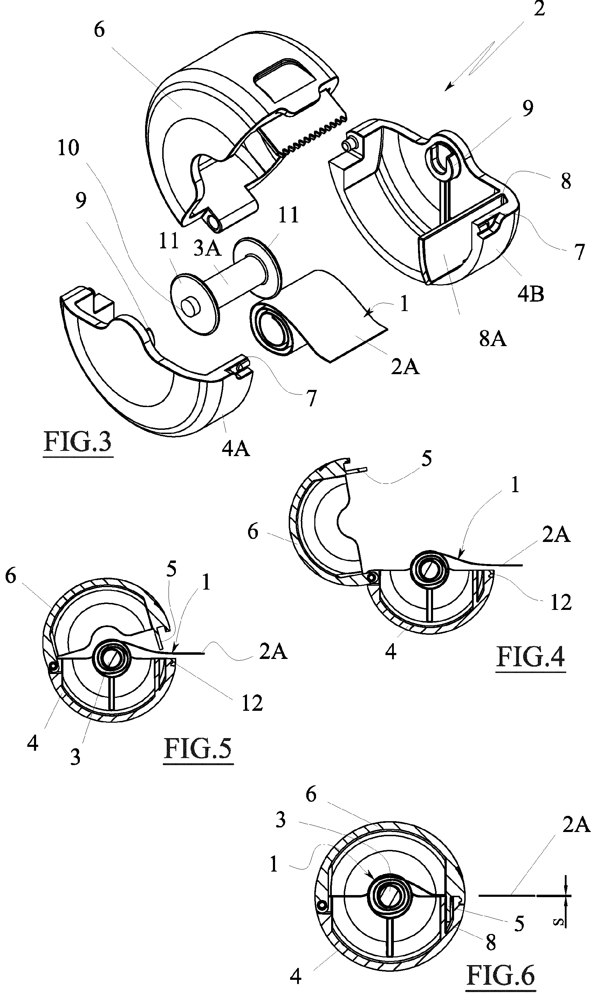

As can be seen in Figure 4 meanwhile, apart from the spool 3 on which the product 1 is wound, the article can comprise just a few pieces. Between such pieces, a base 4 can be envisaged, which is formed of two half-shells 4A, 4B, the said shells mutually engaging so as to constrain the lid 6 in a hinge-like manner.

The cavity 8, meanwhile, can also feature a partition 8A, which extends from just one of the two half-shells, in particular from half-shell 4B.

The article is advantageously made entirely of plastic (e.g. PP, PET, PE, PS), obviously with the exception of the cosmetic product 1, and optionally of the cutter 5 (which can also be made of metal).

However, as alternatives to plastic, other materials can also be used, such as metal, wood, etc.

The cosmetic product 1 (or make-up product) is wound on the spool.

The said cosmetic product is preferably a make-up product such as an eyeshadow, a blush, a bronzer, a face and body highlighter, a lipstick etc.

The only thing that can be wound on the spool is the cosmetic product 1.

In other words, the substance wound on the spool consists entirely and only of a make-up product.

Therefore, the flexible strip 2A (or tape) is made entirely of a make-up product, therefore devoid of a support onto which the make-up product is applied.

As seen, the invention therefore provides the following advantages:
- Ease of use: measured dispensing of the cosmetic product 1: only the amount of actually product needed for make-up purposes is dispensed and cut;
- Refillable: only the spool on which the product is wound is replaced, while the external part of the packaging is reused;
- Since the cosmetic product 1 mat feature a "water-activated" texture, the method for dispensing the product prevents exposure to moisture since only the part that will be used is moistened (for activation thereof).

The cosmetic product 1 (or the make-up product) formed as a flexible strip 2A, can have a flexible solid form and can comprise a blend of cosmetic powders and the following:
a) 0.1-15% by weight of at least one carrageenan with respect to the cosmetic product.

Carrageenans are a family of compounds of polysaccharide nature (CAS Number: 9000-07-1) obtained by boiling red algae from the rocky coast of the North Atlantic (Chondruscrispus and Gigartinamamitiosa, also known as Irish moss, marine lichen or carragheen).

There are mainly three types of carrageenans: kappa (k-), iota (i-) and lambda (1-), which differ from one another in the number and location of the sulphate groups and the bond that joins the individual monomers. As a rule, the greater the number of sulphate groups, the lower the viscosity capacity.

For the objects of the present invention, iota (i-) carrageenans are preferred, due to their ability to produce soft, extremely elastic gels. The addition of glucose and potassium chloride increases this characteristic.

Kappa (k-) carrageenans can also be used, as their characteristic is that they stabilise the formulation and improve the texture of the product.

Preferably, at least two different carrageenans are present, each one having a different viscosity capacity.

There are various commercial carrageenans available, each one having specific viscosities and properties, and such carrageenans can be used together in the composition of the product according to the invention. Particularly preferable are the following carrageenans: Safic care t ck-1 marketed by Safic-alcan, carrageenan iota by Flower Tales Cosmetics, and GENUVISCO^{®} CG-131, carrageenan iota by CP Kelco.

Depending on the make-up product in question, the term "cosmetic powder blend" refers to a blend of solid powder ingredients of which such make-up products are typically constituted. For example, cosmetic powders may include ingredients such as Talc, Mica, Bismuth Chloride Oxide (CI77163), Iron Oxides (CI77491-2-9), Ultramarine Blue (CI77007), Manganese Violet (CI77742), Chromium Hydroxide (CI77289), Chromium Oxide (CI77288), Ferric Ammonium Ferrocyanide (CI77510), Titanium Dioxide (CI77891), D&C Red 7 CA Lake, D&C Red 19 Al Lake, D&C Red 6 Ba Lake, D&C Red 3 Al Lake, D&C Red 9 BA Lake, D&C Red 21 Al Lake, D&C Yellow 5 Al Lake, D&C Red 30 Al Lake, D&C Yellow 10 Al Lake, D&C Red 27 Al Lake, D&C Yellow 5 Al Lake, D&C Orange 5, FD&C Yellow 6 Al Lake, FD&C Blue 1 Al Lake, D&C red 36 Al Lake, Carmine (CI75470), Fluorphlogopite (Synthetic Mica), Boron Nitride, Calcium Aluminium Borosilicate, Magnesium Aluminium Borosilicate.

(In the list above, the "CI" number shown in brackets is the Colour Index reference number; for organic lacquers, the CTFA NAME is also included).

Preferably, the cosmetic product 1 envisaged in a flexible solid form comprises a cosmetic powders blend and the following:
a) 0.1-15% by weight of at least one carrageenan with respect to the cosmetic product,
   the said cosmetic product furthermore has at least one of the following technical characteristics:
   - hardness [shore A]: 20 - 60 (ASTM D2240)
   - tensile strength [N/mm²]: 2 - 10 (ASTM D412)
   - elongation [%]: 5-15 (ASTM D412)
   - tear resistance [N/mm]: 10 - 40 (ASTM D624).

Indeed, these technical characteristics (preferably all) ensure the product remains suitably flexible, therefore resistant to impacts and pleasant to the touch, while remaining solid and therefore easy to handle.

More preferably, the product according to the invention comprises 40-60% by weight of a blend of cosmetic powders and the following:
a) 0.5-10% by weight of at least one carrageenan;
b) 0.05-5% by weight of a non-emulsifying ester of choice from isononyl isononanoate, isodecyl neopentanoate, octyldodecyl neopentanoate, isostearyl isostearate, isopropyl myristate, isocetyl stearate, and mixtures thereof;
c) 0.1-5% by weight of methyl glucose dioleate ether;
d) 0.1-5% by weight of two or more of the following:
   glyceryl stearate, behenyl alcohol, palmitic acid, stearic acid, lecithin, lauryl alcohol, myristyl alcohol, and cetyl alcohol;
e) 5-10% by weight of glycerine;
   with respect to the weight of the product.

In preferred embodiments, the said non-emulsifying ester b) is isononyl isononanoate.

In further preferred embodiments, the ingredient d) is a blend of glyceryl stearate, behenyl alcohol, palmitic acid, stearic acid, lecithin, lauryl alcohol, myristyl alcohol, and cetyl alcohol.

The cosmetic product 1 according to the present invention is characterised by a unique texture, which includes several characteristics usually associated with different types of products that have never previously coexisted in a single formula. For example, the said product has a compact appearance and a consistency similar to that of an extrudate when dispensed and a consistency comparable to a poured product when applied to the skin.

Advantageously, the cosmetic product 1 (or make-up product) is inert when dry (i.e. when placed in contact with a dry surface, including the skin, the said product does not create colour or release components).

When moistened however, i.e. placed in contact with polar substances such as water, glycols, or alcohols, the said product immediately has payoff (i.e. immediately releases part of the product, for example colour on the skin upon application).

Thanks to these unique characteristics, the make-up product can be transported and touched without releasing colour undesirably, while one simply has to moisten the said product to render the latter usable for make-up purposes.

As already mentioned, such cosmetic product 1 (or make-up product) is made in the form of a strip 2A which is then wound on the said spool.

For example, the strip 2A wound on the spool can have a width L of between 5 mm and 30 mm, preferably 25 mm, and a thickness S of between 0.5 mm and 1.5 mm, preferably 0.5 mm.

Preferably, the said thickness S is of 0.5 to 1 mm, more preferably greater than or equal to 0.5 mm and less than or equal to 1 mm.

The overall length of the strip 2A wound on the spool 3 can be between 0.4 and 1.5 meters, preferably approximately 0.5 m, in the event that the said strip is formed entirely of make-up product.

The portion of strip 2A dispensed at each use can be indicatively between 1.5 cm and 10 cm in length, preferably 6 cm.

The present invention is therefore also aimed at the use of the said cosmetic article 2 to apply make-up to the skin by means of a method comprising the following steps in series:
- cutting a portion of the cosmetic product 1 off the strip 2A
- moistening the cut portion of the cosmetic product 1 with a polar substance, preferably water, and
- applying the make-up substance released by the thus moistened make-up product to the skin.

When applied to the skin, the make-up product 1 according to the invention forms a perfectly adherent but imperceptible film of make-up on the skin, which also has a long hold.

When dry, the cosmetic product 1 (or make-up product) is inert, deformable (in a essentially elastic manner), and resistant to impacts, and can be made in the form of a very thin strip, which can therefore be easily wound on the spool 3.

The process for preparing the cosmetic product 1 (or make-up product) may include the conventional step of supplying the blend of cosmetic powders, which can be prepared for example according to the disclosures in patent EP2189150 B1, followed by further steps specific to the present process which contribute to the formation of the desired product.

In particular, the process for preparing the product according to the present invention comprises the following steps:
i. supplying a blend of cosmetic powders;
ii. mixing the said cosmetic powders with an emulsion comprising water and at least one carrageenan, preferably in a mixer at a speed of 5-500 rpm, to obtain a paste;
iii. spreading a layer of the said paste, in the form of a strip 2A, onto a solid flat support, for example a tray, the said layer preferably having a thickness of 0.5-1.5 mm;
iv. drying the blend by placing the said support in an oven;
v. remove the thus dried layer of paste from the support, thereby obtaining the cosmetic product in solid form;
vi. optionally, punching or cutting the solid-form cosmetic product, thereby obtaining the strip-form cosmetic product 1;
vii. winding the strip 2A on the spool 3.

In step i., the cosmetic powders are preferably supplied in the form of a blend, i.e. a homogeneous mix, by using a high-speed mixer.

Preferably, step ii. of the process is carried out in a special turboemulsifier.

Preferably, in step ii., the cosmetic powders and the emulsion are mixed in a weight ratio of 10:90 to 50:50, more preferably of 10:90 to 40:60. In preferred embodiments, the cosmetic powders and the emulsion are mixed in a weight ratio of approximately 30:70.

More preferably, in step ii., the said emulsion comprises water and:
a) 0.5-10% by weight of at least one carrageenan;
b) 0.05-5% by weight of a non-emulsifying ester of choice from isononyl isononanoate, isodecyl neopentanoate, octyldodecyl neopentanoate, isostearyl isostearate, isopropyl myristate, isocetyl stearate, and mixtures thereof;
c) 0.1-5% by weight of methyl glucose dioleate ether;
d) 0.1-5% by weight of two or more of the following: glyceryl stearate, behenyl alcohol, palmitic acid, stearic acid, lecithin, lauryl alcohol, myristyl alcohol, and cetyl alcohol;
e) 1-10% by weight of glycerine;
with respect to the weight of the emulsion.

In preferred embodiments, in step ii., the said emulsion comprises water and:
a) 0.5-5% by weight of at least one carrageenan;
b) 0.05-5% by weight of a non-emulsifying ester of choice from isononyl isononanoate, isodecyl neopentanoate, octyldodecyl neopentanoate, isostearyl isostearate, isopropyl myristate, isocetyl stearate, and mixtures thereof;
c) 0.5-3% by weight, preferably approximately 2% by weight, of a methyl glucose dioleate ether, preferably PEG-120 methyl glucose dioleate, such as Glucamate^{™} DOE-120 by Lubrizol;
d) 0.5-3% by weight, preferably approximately 2% by weight, of glyceryl stearate, behenyl alcohol, palmitic acid, stearic acid, lecithin, lauryl alcohol, myristyl alcohol, and cetyl alcohol, such as the mix produced by Ashland and marketed with the trade name ProLipid^{™} 141,
e) 1-10% by weight of glycerine;
with respect to the weight of the emulsion.

Preferably, in step iv., the blend is dried in an oven at a temperature of 25-70°C, more preferably 30-50°C, even more preferably at approximately 40°C for a few hours, preferably for 5-10 hours, even more preferably for approximately 7 hours. Once dried, the solid-form cosmetic product 1 obtained can be further shaped/die-cut into the desired shape, for example circles, diamonds, stars, etc.

The process for preparing the flexible solid cosmetic product is preferably a process for preparing a flexible solid eyeshadow wherein the cosmetic powders provided in step i. are obtained by mixing the constituents of the eyeshadow (such as: colorants, talc, mica, silica, pigments) and sieving to ensure a blend with particle homogeneity.

Preferably, the blend of cosmetic powders comprises pearlescent pigments, typically formed of mica coated with titanium oxide, in a concentration of between 20 and 70% by weight, with respect to the total weight of the blend of powders.

Preferably, the flexible solid cosmetic product 1, obtainable from the preparation process described above, further features at least one of the following technical characteristics:
- hardness [shore A]: 20 - 60 (ASTM D2240)
- tensile strength [N/mm²]: 2 - 10 (ASTM D412)
- elongation [%]: 5-15 (ASTM D412)
- tear resistance [N/mm]: 10 - 40 (ASTM D624).

The combination of all these characteristics is particularly advantageous.

Unlike the compact products typical of the prior art, the product according to the present invention, thus obtained, can be directly placed in a pouch made of a flexible material, such as paper or silicone, without needing to be placed in a rigid godet to prevent breakage, as this product is flexible and resistant to impacts. It can therefore be handled directly, without the need for protective wrappers or containers.

Indeed, as already discussed, the composition of the product and the preparation process allow for the production of a product with a very low thickness D (even less than or equal to 1 mm), advantageously without the need to compact the final product.

To the touch, product 1 seems solid, almost rubbery, and dry, but extremely flexible and resistant at the same time.

As mentioned, as the product is flexible, it is not necessary to contain the product in a rigid primary package, such as a godet, a jar, a bottle, or a tube), as it maintains a high resistance to impact, deformation, and traction (unlike compact, baked or extruded products). This is all to the advantage of the environment.

The extraordinary flexibility of product 1 emerges very clearly upon analysis of the annexed figures. It can be seen that the cosmetic product 1 can be easily wound on a spool 3.

Advantageously, the cylindrical part 3A of the spool where the cosmetic product 1 in strip form is wound can have a diameter of between 5 mm and 20 mm, preferably between 5 and 7 mm (obviously this depends on the flexibility of the strip 2A) .

It has been seen that this size allows excellent winding without damage to the strip 2A.

The product 1 can be folded essentially into a U shape without breaking and without cracks forming on the surface thereof. The bending can be obtained, for example, by applying gentle force (for example with the fingers) to the edges of the cut part of the strip 2A. Once the force on the edges has ceased, the product returns, in an essentially elastic manner, to its original - therefore essentially flat - configuration, as shown in Figure 2.

After bending, only slight or no deformations remain in the product, which however can be levelled out by applying very light pressure with the fingers.

It is possible that prior to cutting, the part of the strip 2A protruding from the base 4 bends a little due to its own weight, taking on a slightly concave, downwards form.

All without breaking, cracking, or releasing dust or traces of product (unless the said product has been moistened) .

Furthermore, unlike cast or cream products, the product has a high resistance to thermal shock and especially to heat.

A further advantage lies in the fact that the product 1 does not release colour unless moistened with a polar solvent, for example with water, or glycols, preferably at least of the pentylene type.

Using water, the product dries on the skin in a very short space of time (from 1 to 3 minutes).

It should also be understood that all aspects identified as favourable and advantageous for the cosmetic product should be deemed equally preferable and advantageous also for the uses and the preparation process thereof, in addition to the product obtainable from the said process.

It should likewise be understood that all combinations of the preferred aspects of the cosmetic product envisaged in the invention, as well as the uses and the preparation process thereof, as set out above, should be considered described herein.

Various embodiments of the innovation have been disclosed herein, but further embodiments may also be conceived using the same innovative concept.

Examples of embodiments of the cosmetic product 1 wound on the spool are provided below for illustrative purposes.

### EXAMPLES

### Example 1.

The cosmetic product 1 prepared is an eyeshadow.

The composition of the starting ingredients is reported below:

| | **Ingredients** | **% by weight (with respect to the total weight of the ingredients)** | **Commercial product** |
|---|---|---|---|
| 1 | CI 77891 (titanium dioxide) | 30.000 | |
| | zinc oxide | | |
| | mica | | |
| 2 | potassium sorbate | 0.275 | |
| 3 | dehydroacetic acid | 0.275 | |
| 4 | isononyl isononanoate | 20.000 | DUB ININ 14031 |
| 5 | glycerin | 4.000 | |
| 6 | tocopherol | 2.000 | GLUCAMATE^{™} DOE-120 |
| | PEG-120 methyl glucose dioleate | | |
| 7 | lauryl alcohol | 2.000 | PROLIPID^{™} 141 |
| | glyceryl stearate | | |
| | cetyl alcohol | | |
| | lecithin | | |
| | palmitic acid | | |
| | myristyl alcohol | | |
| | behenyl alcohol | | |
| | acid stearic | | |
| 8 | *ChondrusCrispus* | 1.500 | SAFIC' CARE T CK1 |
| | glucose | | |
| | potassium chloride | | |
| 9 | *ChondrusCrispus powder* | 1.000 | GENUVISCO^{®} CARRAGEENAN CG-131 |
| 10 | water | 38.950 | |

where:
- Safic care T CK-1, by Safic-alcan, comprising iota-carrageenan, glucose, and potassium chloride;
- GENUVISCO^{®} CG-131, by CP Kelco, iota-carrageenan;
- Glucamate^{™} DOE-120, by Lubrizol, as methyl ether glucose dioleate;
- ProLipid^{™} 141, by Ashland, as a mixture of glyceryl stearate, behenyl alcohol, palmitic acid, stearic acid, lecithin, lauryl alcohol, myristyl alcohol cetyl alcohol. ProLipid^{™} 141 is used as a moisturising agent and to improve glidability.

First, a powder blend is supplied comprising ingredients 1 to 3. This blend is made in a standard mill. The pearlescent elements are added at the end of the process to maintain the properties thereof.

An emulsion comprising ingredients 4 to 10 is made separately using a turbo emulsifier.

Subsequently, the powder blend and the emulsion are mixed using a Conti PL760EV 300 mixer at 300 rpm for a length of time varying from approximately 5 to 10 minutes.

The resulting paste (bulk) is spread evenly over a steel or plastic tray with a height of approximately 2 mm, to form a strip measuring approximately 2 m in length.

The tray is kept in the oven at approximately 40°C for approximately 7 h to facilitate evaporation of the volatile part of the emulsion.

After baking, the dry bulk is removed from the tray as a flexible strip with a thickness of about 1 mm (due to shrinkage), which is then shaped/die-cut lengthwise to about 0.5 m and possibly widthwise, and wound on the spool 3.

The final composition of the eyeshadow product is as follows:

| | **Ingredients** | **% by weight (with respect to the total weight of the ingredients)** |
|---|---|---|
| 1 | CI 77891 (titanium dioxide) | 49.898 |
| | zinc oxide | |
| | mica | |
| 2 | potassium sorbate | 0.446 |
| 3 | dehydroacetic acid | 0.446 |
| 4 | isononyl isononanoate | 33.200 |
| 5 | glycerin | 6.480 |
| 6 | tocopherol | 3.240 |
| | PEG-120 methyl glucose dioleate | |
| 7 | lauryl alcohol | 3.240 |
| | glyceryl stearate | |
| | cetyl alcohol | |
| | lecithin | |
| | palmitic acid | |
| | myristyl alcohol | |
| | behenyl alcohol | |
| | acid stearic | |
| 8 | *ChondrusCrispus* | 2.430 |
| | glucose | |
| | potassium chloride | |
| 9 | *ChondrusCrispus powder* | 1.620 |

### Example 2.

The cosmetic product 1 is an eyeshadow.

First of all, a powder blend is provided, comprising:

| **Ingredients** | **% by weight (of the total weight of the blend)** |
|---|---|
| Sodium dehydroacetate | 0,3 |
| Sorbic acid | 0,4 |
| Magnesium myristate | 3 |
| Zinc laurate | 3 |
| Zinc stearate | 0,9 |
| Talc | 30,4 |
| Silica | 2 |
| Mica pigments | 40 |
| Pigments | 20 |

This blend is made in a standard mill. The pearlescent elements are added at the end of the process to maintain the properties thereof.

An emulsion comprising ingredients 4 to 10 stated in the table in Example 1 is made separately using a turbo emulsifier.

Subsequently, the powder blend and the emulsion are mixed using a Conti PL760EV 300 mixer at 300 rpm for a length of time varying from approximately 5 to 10 minutes.

The resulting paste (bulk) is spread evenly, in a strip shape, over a steel tray with a height of approximately 2 mm.

The tray is kept in the oven at approximately 40°C for approximately 7 h to facilitate evaporation of the volatile part of the emulsion.

Once baked, the dry bulk is removed from the tray as an approximately 1 mm-thick strip which is shaped/die-cut to bring the bulk to a length of 0.5 m and to better delimit the lateral edges thereof, and then wound on the spool 3.

## Claims

1. Cosmetic article (2) comprising a cosmetic product (1), preferably a makeup product, in the form of a flexible strip (2A) rolled on a spool (3), a base (4) that rotatably supports the spool (3), and a lid (6) that, when in a closed position, protects the spool (3) on which the cosmetic product (1) is rolled, **characterized in that** the strip (2A) wound on the spool is completely made of a make-up product.

2. Article according to claim 1, wherein the article comprises a cutter (5) for cutting part of the strip (2A) of cosmetic product (1).

3. Article according to the preceding claim, wherein the cutter (5) is associated with the lid (6).

4. Article according to claim 1, wherein the base (4) defines a plane (7) to which an end of the strip (2A) is rested.

5. Article according to the preceding claim, wherein the base, between the plane (7) and the spool (3) defines a cavity (8) substantially conformed as said cutter (5), wherein said cutter (5) is inserted to cut said strip (2A) when the strip (2A) is resting on the plane (7) so as to protrude from the base (4), and the lid (6) is closed.

6. Article according to claim 1, wherein the lid (6) is hinged to the base (4).

7. Article according to claim 1, wherein the spool is removably associated with the base (4) so that it can be replaced or refilled once the original spool is empty.

8. Article according to one or more of the preceding claims, wherein the base (4) defines at least one pair of cradle seats (9) housing corresponding pins (10) extending from the spool (3), wherein said pins (10) provide, in the vicinity of each of said pins (10) a flange (11) laterally containing the strip (2A).

9. Article according to one or more of the preceding claims, wherein between the lid (6) and the base (4) are provided releasable snap means (12) configured to keep the lid (6) closed at least when the cosmetic article (1) is not in use.

10. Article according to claim 1, wherein the cosmetic product (1) in a flexible solid form, shaped as a strip (2A), comprises a mixture of cosmetic powders and:
(a) 0.1-15% by weight of at least one carrageenan, on the weight of the cosmetic product.

11. Article according to claim 10, wherein said at least one carrageenan is carrageenan-iota.

12. Article according to claim 10, wherein said cosmetic product (1) further has at least one of the following technical characteristics:
- hardness [shore A]: 20 - 60 (ASTM D2240)
- tensile strength [N/mm2]: 2 - 10 (ASTM D412)
- elongation [%]: 5 - 15 (ASTM D412)
- tear strength [N/mm]: 10 - 40 (ASTM D624).

13. Article according to claim 10, wherein the cosmetic product (1) comprises 40-60% by weight of cosmetic powder mixture and:
(a) 0.5-10% by weight of at least one carrageenan;
(b) 0.05-5% by weight of a non-emulsifying ester chosen from isononyl isononanoate, isodecylneopentanoate, octyldodecylneopentanoate, isostearylisostearate, isopropyl myristate, isocetyl stearate, and mixtures thereof;
(c) 0.1-5% by weight of methyl glucose dioleateether;
(d) 0.1-5% by weight of two or more of Glyceryl Stearate, Behenyl Alcohol, Palmitic Acid, Stearic Acid, Lecithin, Lauryl Alcohol, Myristyl Alcohol, and CetylAlcohol;
(e) 5-10% by weight of glycerin;
by weight of the product.

14. Method for making up the skin through an article according to one or more of the preceding claims, comprising the following steps:
- cutting a part of the strip (2A)
- moistening the cut part strip (2A) with a polar substance, preferably water, and
- apply said moistened product to the skin.

## Patentansprüche

1. Kosmetikartikel (2), umfassend ein Kosmetikprodukt (1), bevorzugt ein Schminkprodukt, in der Form eines flexiblen Streifens (2A), der auf einer Spule (3) aufgerollt ist, eine Basis (4), welche die Spule (3) drehbar stützt, und einen Deckel (6), der, wenn er in einer geschlossenen Position ist, die Spule (3), auf der das Kosmetikprodukt (1) aufgerollt ist, schützt, **dadurch gekennzeichnet, dass** der Streifen (2A), der auf der Spule aufgewickelt ist, vollständig aus einem Schminkprodukt hergestellt ist.

2. Artikel nach Anspruch 1, wobei der Artikel ein Schneidwerkzeug (5) zum Schneiden von Teil des Streifens (2A) aus Kosmetikprodukt (1) umfasst.

3. Artikel nach dem vorhergehenden Anspruch, wobei das Schneidwerkzeug (5) mit dem Deckel (6) assoziiert ist.

4. Artikel nach Anspruch 1, wobei die Basis (4) eine Ebene (7) definiert, an der ein Ende des Streifens (2A) anliegt.

5. Artikel nach dem vorhergehenden Anspruch, wobei die Basis zwischen der Ebene (7) und der Spule (3) einen Hohlraum (8) definiert, der im Wesentlichen als das Schneidwerkzeug (5) angepasst ist, wobei das Schneidwerkzeug (5) eingesetzt ist, um den Streifen (2A) zu schneiden, wenn der Streifen (2A) auf der Ebene (7) aufliegt, um von der Basis (4) vorzustehen, und der Deckel (6) geschlossen ist.

6. Artikel nach Anspruch 1, wobei der Deckel (6) an der Basis (4) angelenkt ist.

7. Artikel nach Anspruch 1, wobei die Spule entfernbar mit der Basis (4) assoziiert ist, sodass sie ersetzt oder nachgefüllt werden kann, sobald die ursprüngliche Spule leer ist.

8. Artikel nach einem oder mehreren der vorhergehenden Ansprüche, wobei die Basis (4) zumindest ein Paar von Wiegesitzen (9) definiert, die entsprechende Stifte (10) aufnehmen, die sich von der Spule (3) erstrecken, wobei die Stifte (10) in der Nähe von jedem der Stifte (10) einen Flansch (11) bereitstellen, der den Streifen (2A) lateral enthält.

9. Artikel nach einem oder mehreren der vorhergehenden Ansprüche, wobei zwischen dem Deckel (6) und der Basis (4) lösbare Schnappmittel (12) bereitgestellt sind, die dazu konfiguriert sind, den Deckel (6) geschlossen zu halten, zumindest wenn der Kosmetikartikel (1) nicht in Verwendung ist.

10. Artikel nach Anspruch 1, wobei das Kosmetikprodukt (1) in einer flexiblen festen Form, geformt als ein Streifen (2A), eine Mischung aus Kosmetikpulvern und Folgendem umfasst:
(a) 0,1-15 Gewichts-% von zumindest einem Carrageen bezogen auf das Gewicht des Kosmetikprodukts.

11. Artikel nach Anspruch 10, wobei das zumindest eine Carrageen Carrageen-iota ist.

12. Artikel nach Anspruch 10, wobei das Kosmetikprodukt (1) ferner zumindest eine der folgenden technischen Eigenschaften aufweist:
- Härte [Shore A]: 20 - 60 (ASTM D2240),
- Zugfestigkeit [N/mm2]: 2 - 10 (ASTM D412),
- Dehnung [%]: 5 - 15 (ASTM D412),
- Reißfestigkeit [N/mm]: 10 - 40 (ASTM D624).

13. Artikel nach Anspruch 10, wobei das Kosmetikprodukt (1) 40-60 Gewichts-% Kosmetikpulvermischung und Folgendes umfasst:
(a) 0,5-10 Gewichts-% von zumindest einem Carrageen;
(b) 0,05-5 Gewichts-% eines nicht emulgierenden Esters gewählt aus Isononylisononanoat, Isodecylneopentanoat, Octyldodecylneopentanoat, Isostearylisostearat, Isopropylmyristat, Isocetylstearat und Mischungen davon;
(c) 0,1-5 Gewichts-% Methylglucosedioleatether;
(d) 0,1-5 Gewichts-% von zwei oder mehr von Glycerylstearat, Behenylalkohol, Palmitinsäure, Stearinsäure, Lecithin, Laurylalkohol, Myristylalkohol und Cetylalkohol;
(e) 5-10 Gewichts-% Glycerin;
nach Gewicht des Produkts.

14. Verfahren zum Schminken der Haut durch einen Artikel nach einem oder mehreren der vorhergehenden Ansprüche, umfassend die folgenden Schritte:
- Schneiden eines Teils des Streifens (2A),
- Anfeuchten des geschnittenen Teilstreifens (2A) mit einer polaren Substanz, bevorzugt Wasser, und
- Auftragen des angefeuchteten Produkts auf die Haut.

## Revendications

1. Article cosmétique (2) comprenant un produit cosmétique (1), de préférence un produit de maquillage, sous la forme d'une bande souple (2A) enroulée sur une bobine (3), une base (4) qui supporte de manière rotative la bobine (3), et un couvercle (6) qui, lorsqu'il est en position fermée, protège la bobine (3) sur laquelle le produit cosmétique (1) est enroulé, **caractérisé en ce que** la bande (2A) enroulée sur la bobine est entièrement constituée d'un produit de maquillage.

2. Article selon la revendication 1, dans lequel l'article comprend un couteau (5) pour couper une partie de la bande (2A) de produit cosmétique (1).

3. Article selon la revendication précédente, dans lequel le couteau (5) est associé au couvercle (6).

4. Article selon la revendication 1, dans lequel la base (4) définit un plan (7) sur lequel une extrémité de la bande (2A) repose.

5. Article selon la revendication précédente, dans lequel la base, entre le plan (7) et la bobine (3) définit une cavité (8) sensiblement conforme audit couteau (5), dans lequel ledit couteau (5) est inséré pour couper ladite bande (2A) lorsque la bande (2A) repose sur le plan (7) de manière à faire saillie à partir de la base (4), et le couvercle (6) est fermé.

6. Article selon la revendication 1, dans lequel le couvercle (6) est articulé sur la base (4) .

7. Article selon la revendication 1, dans lequel la bobine est associée de manière amovible à la base (4) de sorte qu'elle puisse être remplacée ou rechargée une fois que la bobine d'origine est vide.

8. Article selon une ou plusieurs des revendications précédentes, dans lequel la base (4) définit au moins une paire de sièges de berceau (9) logeant des broches correspondantes (10) s'étendant à partir de la bobine (3), dans lequel lesdites broches (10) fournissent, à proximité de chacune desdites broches (10), une bride (11) contenant latéralement la bande (2A) .

9. Article selon une ou plusieurs des revendications précédentes, dans lequel entre le couvercle (6) et la base (4) sont prévus des moyens d'encliquetage libérables (12) configurés pour maintenir le couvercle (6) fermé au moins lorsque l'article cosmétique (1) n'est pas utilisé.

10. Article selon la revendication 1, dans lequel le produit cosmétique (1) sous une forme pleine souple, en forme de bande (2A), comprend un mélange de poudres cosmétiques et :
(a) 0,1 à 15 % en poids d'au moins un carraghénane, par rapport au poids du produit cosmétique.

11. Article selon la revendication 10, dans lequel ledit au moins un carraghénane est le carraghénane-iota.

12. Article selon la revendication 10, dans lequel ledit produit cosmétique (1) présente en outre au moins l'une des caractéristiques techniques suivantes :
- dureté [Shore A] : 20 à 60 (ASTM D2240)
- résistance à la traction [N/mm2] : 2 à 10 (ASTM D412)
- allongement [%] : 5 à 15 (ASTM D412)
- résistance à la déchirure [N/mm] : 10 à 40 (ASTM D624).

13. Article selon la revendication 10, dans lequel le produit cosmétique (1) comprend 40 à 60 % en poids de mélange de poudre cosmétique et :
(a) 0,5 à 10 % en poids d'au moins un carraghénane ;
(b) 0,05 à 5 % en poids d'un ester non émulsifiant
choisi parmi l'isononanoate d'isononyle, le néopentanoate d'isodécyle, le néopentanoate d'octyldodécyle, l'isostéarylisostéarate, le myristate d'isopropyle, le stéarate d'isocétyle et leurs mélanges ;
(c) 0,1 à 5 % en poids de dioléateéther de méthylglucose ;
(d) 0,1 à 5 % en poids de deux ou plus de stéarate de glycéryle, d'alcool béhénylique, d'acide palmitique, d'acide stéarique, de lécithine, d'alcool laurique, d'alcool myristylique et d'alcool cétylique ;
(e) 5 à 10 % en poids de glycérine ;
en poids du produit.

14. Procédé de maquillage de la peau à l'aide d'un article selon une ou plusieurs des revendications précédentes, comprenant les étapes suivantes :
- découpe d'une partie de la bande (2A)
- humidification la bande de partie coupée (2A) avec une substance polaire, de préférence de l'eau, et
- application dudit produit humidifié sur la peau.
